(19) Europäisches Patentamt
European Patent Office
Office européen des brevets

(11) Veröffentlichungsnummer: **0 289 451 B1**

(12) **EUROPÄISCHE PATENTSCHRIFT**

(45) Veröffentlichungstag der Patentschrift: **27.10.93**

(51) Int. Cl.$^5$: **C07J 1/00**, C07J 31/00, A61K 31/565

(21) Anmeldenummer: **88730102.6**

(22) Anmeldetag: **29.04.88**

# (54) 3-Methylen-4-androsten-17-one, Verfahren zu deren Herstellung und diese enthaltende pharmazeutische Präparate.

(30) Priorität: **30.04.87 DE 3714965**

(43) Veröffentlichungstag der Anmeldung:
**02.11.88 Patentblatt 88/44**

(45) Bekanntmachung des Hinweises auf die Patenterteilung:
**27.10.93 Patentblatt 93/43**

(84) Benannte Vertragsstaaten:
**AT BE CH DE ES FR GB GR IT LI LU NL SE**

(56) Entgegenhaltungen:
**EP-A- 0 100 566**
**EP-A- 0 161 492**

**CHEMICAL ABSTRACTS, Band 105, Nr. 9, 01 September 1986, Columbus, OH (US); S.MIYAIRI et al., Seiten 87-88, Nr. 72831f&NUM;**

(73) Patentinhaber: **SCHERING AKTIENGESELLSCHAFT Berlin und Bergkamen**
**Müllerstrasse 170/178**
**Postfach 65 03 11**
**D-13303 Berlin(DE)**

(72) Erfinder: **Bohlmann, Rolf, Dr.**
**Melanchtonstrasse 23**
**D-1000 Berlin 21(DE)**
Erfinder: **Laurent, Henry, Dr.**
**Glambecker Weg 21**
**D-1000 Berlin 28(DE)**
Erfinder: **Hendersen, David, Dr.**
**Jahnstrasse 17**
**D-1000 Berlin 28(DE)**
Erfinder: **Nishino, Yukishige, Dr.**
**Lanzendorfer Weg 14**
**D-1000 Berlin 22(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

**Beschreibung**

Die Erfindung betrifft 3-Methylen-4-androsten-17-one, Verfahren zu deren Herstellung und diese enthaltende pharmazeutische Präparate.

Die erfindungsgemäßen Verbindungen werden durch die allgemeine Formel I beschrieben:

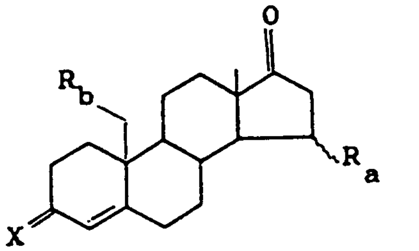

(I),

worin

$R_a$ ein Wasserstoffatom oder einen gesättigten oder ungesättigten, gerad- oder verzweigtkettigen, gegebenenfalls substituierten Kohlenwasserstoffrest mit 1 - 6 Kohlenstoffatomen, wobei $R_a$ in der $\alpha$- oder $\beta$-Position steht, und

$R_b$ ein Wasserstoffatom, eine Hydroxyl- oder eine $-S(O)_nR_c$-Gruppe, bei der $R_c$ ein Wasserstoffatom oder eine Acylgruppe mit 1 - 4 Kohlenstoffatomen und n = 0 oder $R_c$ eine Alkylgruppe mit 1-4 Kohlenstoffatomen und n = 0, 1 oder 2 ist, und

X $CH_2$, CHF, CHCl oder CHBr

bedeuten, wobei X nicht $CH_2$ ist, wenn $R_a$ Wasserstoff und $R_b$ Hydroxyl oder $R_a$ und $R_b$ jeweils Wasserstoff bedeuten.

Die für $R_a$ stehenden Alkylgruppen sollen gerad- oder verzweigtkettig, gesättigt oder ungesättigt, substituiert oder unsubstituiert sein und 1 - 6 Kohlenstoffatome enthalten. Diese Alkylgruppen können am Steroidgerüst $\alpha$- oder $\beta$-ständig sein. Bevorzugt sind die Methyl-, Ethyl-, Propyl-, Butyl-, Hexyl-, 2-Methylpropyl-, 3-Methylbutyl-, Ethenyl-, Hydroxymethyl-, 2-Chlorethyl-, 1-Acetoxyethyl- und 1,2-Bis-(acetoxy)butylgruppe.

Die für $R_b$ stehenden Gruppen können ein Wasserstoffatom, eine Hydroxyl- oder eine $-S(O)_nR_c$Gruppe sein, bei der $R_c$ ein Wasserstoffatom oder eine Alkyl- oder Acylgruppe mit 1 - 4 Kohlenstoffatomen, zum Beispiel eine Methylthio-, Ethylthio-, Propylsulfinyl-, Butylsulfonyl- oder eine Acetylthiogruppe darstellt. Die in 19-Stellung stehende Schwefelfunktionalität kann als Thioether, Sulfoxid, Sulfon oder Acylthiorest vorliegen.

Die erfindungsgemäßen Verbindungen der allgemeinen Formel I

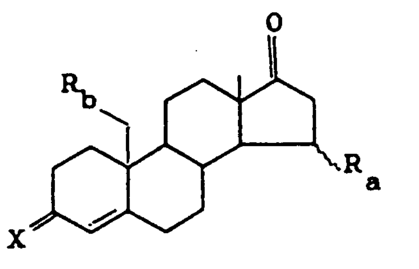

(I),

worin

$R_a$ ein Wasserstoffatom oder einen gesättigten oder ungesättigten, gerad- oder verzweigtkettigen, gegebenenfalls substituierten Kohlenwasserstoffrest mit 1 - 6 Kohlenstoffatomen, wobei $R_a$ in der $\alpha$- oder $\beta$-Position steht, und

$R_b$ ein Wasserstoffatom, eine Hydroxyl- oder eine $-S(O)_nR_c$-Gruppe, bei der $R_c$ ein Wasserstoffatom oder eine Acylgruppe mit 1 - 4 Kohlenstoffatomen und n = 0 oder $R_c$ eine Alkylgruppe mit 1-4 Kohlenstoffatomen und n = 0, 1 oder 2 ist, und

X $CH_2$, CHF, CHCl oder CHBr

bedeuten, wobei X nicht $CH_2$ ist, wenn $R_a$ Wasserstoff und $R_b$ Hydroxyl oder $R_a$ und $R_b$ jeweils Wasserstoff bedeuten,
können hergestellt werden, indem man
eine Verbindung der allgemeinen Formel II

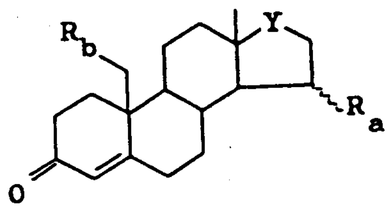

(II),

worin

- $R_a$ ein Wasserstoffatom oder einen gesättigten oder ungesättigten, gerad- oder verzweigtkettigen, gegebenenfalls substituierten Kohlenwasserstoffrest mit 1 - 6 Kohlenstoffatomen, wobei $R_a$ in der $\alpha$- oder $\beta$-Position steht,
- $R_b$ ein Wasserstoffatom, eine Hydroxyl- oder eine $-S(O)_nR_c$-Gruppe, bei der $R_c$ ein Wasserstoffatom oder eine Acylgruppe mit 1 - 4 Kohlenstoffatomen und n =0 oder $R_c$ eine Alkylgruppe mit 1-4 Kohlenstoffatomen und n = 0, 1 oder 2 darstellt und
- Y eine Carbonyl- oder Hydroxymethylengruppe

bedeuten,
mit einem Phosphorylid der allgemeinen Formel III

$(R_d)_3P = X$ (III),

worin

- X $CH_2$, CHF, CHCl oder CHBr, wobei X nicht $CH_2$ ist, wenn $R_a$ Wasserstoff und $R_b$ Hydroxyl oder $R_a$ und $R_b$ Wasserstoff bedeuten, und
- $R_d$ einen gesättigten oder ungesättigten oder aromatischen Kohlenwasserstoffrest mit 1 - 10 Kohlenstoffatomen

bedeuten,
umsetzt und gegebenenfalls oxidiert.

Die Herstellung der Verbindungen der allgemeinen Formel III erfolgt mittels Reaktionen, die dem Fachmann bekannt sind (siehe zum Beispiel Pure Appl. Chem. 1980, 52, 771; Top. Stereochem. 1970, 5, 1; A.W. Johnson, "Ylid-Chemistry" in Academic Press (1966) und Europäische Patentanmeldung Veröffentlichungs-Nr. 0 161 492). Das Triphenylphosphoniumfluormethylen-Wittig-Reagenz wird wie in J. Fluorine Chem. 1985, 27, 85, beschrieben hergestellt. Die analogen Chlor- und Bromderivate sind im Handel erhältlich.

Die Oxidation von Verbindungen der allgemeinen Formel II zu Verbindungen der allgemeinen Formel I wird gegebenenfalls nach Oppenauer (Org. React. 1951, 6, 207) durchgeführt. Außerdem ist es möglich, einen Thioether oder ein Sulfoxid im Rest $R_b$ der allgemeinen Formel II mit bekannten Methoden wahlweise zum Sulfoxid und Sulfon oder zum Sulfon zu oxidieren.

Als literaturbekannte Methoden für diese Oxidationen sind Umsetzungen mit Wasserstoffperoxid, organischen Persäuren, z.B. Chlorperbenzoesäure, und anorganischen Peroxiden, z.B. Nickel- oder Molybdänperoxid, zu nennen [Arch. Pharm. (Weinheim) 316, 941, 1983].

Die Verbindungen der allgemeinen Formel I sind Inhibitoren der Östrogenbiosynthese (Aromatasehemmer). Sie sind damit zur Behandlung von Krankheiten geeignet, die durch Östrogene bedingt oder von Östrogenen abhängig sind. So sind sie geeignet zur Behandlung von östrogeninduzierten oder -stimulierten Tumoren, wie zum Beispiel Mammakarzinom oder Prostatahyperplasie (The Lancet, 1984, 1237-1239).

Der erfindungsgemäßen Verbindungen sind auch wertvoll zur Beeinflussung der Fertilität. So kann eine männliche Infertilität, die aus erhöhten Östrogenspiegeln resultiert, mit den neuen Wirkstoffen behoben werden.

Ferner können die Verbindungen bei Frauen im fortpflanzungsfähigen Alter als Antifertilitätsmittel verwendet werden, um Ovulation oder Implantation durch Östrogenentzug zu hemmen.

Wahrscheinlich sind Aromatasehemmer auch zur Behandlung des drohenden Herzinfarkts geeignet, da erhöhte Östrogenspiegel beim Mann einem Herzinfarkt vorangehen können (US-Patent 4,289,762).

Bekannte, eine die Aromatase hemmende Wirkung aufweisende Steroidsubstanzen sind beispielsweise 4-Hydroxy-androst-4-en-3,17-dion und Ester davon (siehe zum Beispiel US-Patent 4,235,983) und 3-Methylen-4-androsten-17-on (Europäische Patentanmeldung Nr. 0 161 492).

Im Vergleich zu den bekannten Aromatasehemmern wird die Serum-Östradiolkonzentration mit den erfindungsgemäßen Verbindungen bei oraler Applikation stärker gesenkt. Der folgende Test zeigt die Wirksamkeit der erfindungsgemäßen Verbindungen an der Ratte [J. Steroid. Biochem., 7 (1976) 787].

Infantile weibliche Ratten reagieren auf PMSG (Pregnant Mare's Serum Gonadotropin)-Behandlung mit einheitlich erhöhter Steroidsynthese. Die Aromataseaktivität kann durch die Beeinflussung der PMSG-stimulierten Östrogenbildung gemessen werden.

Im sogenannten PMSG-Test werden 21 Tage alte weibliche Ratten alle 2 Tage insgesamt 7 mal mit je 100 I.U. PMSG subkutan vorbehandelt. 1 Stunde vor und 8 Stunden nach der letzten PMSG-Applikation ($d_{12}$) erhalten die Tiere die Testsubstanz durch orale Gabe. 24 Stunden nach der letzten PMSG-Applikation werden die Tiere getötet. Im Serum wird Östradiol radioimmunologisch geprüft. Für jede Gruppe von 10 Tieren wird der Mittelwert der Östradiolkonzentration in nMol/1 mit der Standardabweichung errechnet. Die Signifikanzen der Unterschiede zur Kontrollgruppe werden durch die Varianzanalyse geprüft.

Es wird die prozentuale Hemmung gegen die PMSG-Kontrolle berechnet.

Die literaturbekannten Aromatasehemmer 4-Hydroxy-4-androsten-3,17-dion (A) und 3-Methylen-4-androsten-17-on (B) zeigen im geprüften Dosisbereich (2 x 10 mg) bei oraler Applikation keine Wirksamkeit. Die erfindungsgemäße Verbindung 3-Fluormethylen-19-methylthio-4-androsten-17-on (C) zeigt dagegen im Test mit 45 % eine deutliche Hemmwirkung.

**Tabelle**

PMSG-Test bei infantilen Ratten, oral

| | Dosis mg/Tier 2 x p.o. | Konzentration im Serum Östradiol nMol/l | % Hemmung |
|---|---|---|---|
| PMSG-Kontrolle | - | 6,21 ± 1,78 | - |
| A | 10 | 6,08 ± 0,56 | 0 |
| B | 10 | 6,20 ± 0,80 | 0 |
| C | 10 | 3,42 ± 0,81 | 45 |

Die zu verabreichende Menge der Verbindung schwankt innerhalb eines weiten Bereichs und kann jede wirksame Menge abdecken. In Abhängigkeit des zu behandelnden Zustands und der Art der Verabreichung kann die Menge der verabreichten Verbindung 0,001 - 100 mg/kg Körpergewicht, vorzugsweise 0,01 - 20 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten. Die einzelnen Dosierungseinheiten für die orale Applikation können beispielsweise 10 - 100 mg des Wirkstoffs (Aromataseinhibitors) enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier-oder Emulgiermittels verwendet. Verwendete Öle sind zum Beispiel Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone, wie zum Beispiel Silikonkautschuk. Die Wirkstoffe können außerdem zur perkutanen Applikation zum Beispiel in ein Pflaster eingearbeitet werden.

Die Erfindung betrifft somit auch pharmazeutische Präparate und die Verwendung der neuen Verbindungen der allgemeinen Formel I zur Herstellung dieser Präparate zur Behandlung von östrogen-bedingten Krankheiten.

**Herstellung der Ausgangsverbindungen**

1.) 15α-Ethyl-19-hydroxy-4-androsten-3,17-dion

a) 50,3 g 3β-Hydroxy-5,15-androstadien-17-on [R.W. Kelly and P.J. Sykes, J. Chem. Soc. (C), 416 (1968)] werden in 1,06 l Tetrahydrofuran mit 60,8 g Kaliumcyanid in 350 ml Wasser versetzt und 15 Minuten am Rückfluß gekocht. Dann wird mit Wasser verdünnt und zweimal mit Essigester extrahiert. Man erhält 53 g 15β-Cyano-3β-hydroxy-5-androsten-17-on als Rohprodukt.

b) 22,8 g 15β-Cyano-3β-hydroxy-5-androsten-17-on werden in 600 ml Methanol und 300 ml Tetrahydrofuran mit 11,5 g Natriumborhydrid und 46 ml Wasser portionsweise versetzt und 15 Minuten bei 20 °C gerührt. Dann wird in 10 l Eiswasser gegossen, der Niederschlag abgesaugt, mit Wasser neutral gewaschen und im Trockenschrank bei 70 °C über Nacht getrocknet. Das Rohprodukt wird aus Aceton/Toluol (1:1) kristallisiert. Man erhält 20,6 g 15β-Cyano-5-androsten-3β,17β-diol vom Schmelzpunkt 251 - 255 °C.

c) 18,6 g 15β-Cyano-androsten-3β,17β-diol werden in 700 ml Tetrahydrofuran unter Argon mit 36 g Kalium-tert.-butylat in 300 ml Tetrahydrofuran 15 Minuten am Rückfluß gekocht. Beim Abkühlen kristallisiert das Reaktionsprodukt aus. Durch Absaugen der Kristalle werden 16,4 g 15α-Cyano-5-androsten-3β,17β-diol erhalten.

d) 5 g 15α-Cyano-5-androsten-3β,17β-diol werden in 200 ml Tetrahydrofuran bei -15 °C mit 150 ml Diisobutylalumiumhydridlösung (1,0 M in Tetrahydrofuran) tropfenweise versetzt und 2 Stunden bei 0 °C nachgerührt. Dann werden 300 ml 10 % wäßrige Weinsäure portionsweise zugegeben, in 400 ml Wasser und 200 g Eis eingefällt, abgesaugt, mit Wasser gewaschen und getrocknet. Nach der Kristallisation aus Aceton erhält man 4,0 g 3β,17β-Dihydroxy-5-androsten-15α-carbaldehyd vom Schmelzpunkt 171 - 173 °C (Zersetzung).

e) 19,7 g 3β,17β-Dihydroxy-5-androsten-15α-carbaldehyd werden bei 20 °C zu einer Mischung von 56 g Triphenylmethylphosphoniumbromid in 490 ml Dimethylsulfoxid und 17,6 g Kalium-tert.-butylat gegeben und 2 Stunden bei 20 °C gerührt. Dann wird mit verdünnter Schwefelsäure gefällt und zweimal mit Essigester extrahiert. Die vereinigte organische Phase wird mit Wasser und Natriumchloridlösung gewaschen, über Natriumsulfat getrocknet und im Vakuum eingeengt. Das erhaltene Produkt wird in 500 ml 0,2 n methanolischer Kaliumhydroxid-Lösung 3,5 Stunden bei 20 °C gerührt. Danach wird mit Essigsäure neutralisiert, mit Eiswasser gefällt, mit Wasser gewaschen und im Vakuum bei 70 °C eingeengt. Man erhält 32 g rohes 15α-Vinyl-5-androsten-3β,17β-diol, das mit Triphenylphosphin verunreinigt ist.

f) 32 g rohes 15α-Vinyl-5-androsten-3β,17β-diol werden in 450 ml Methanol mit 3,2 g Palladium (10 %) auf Aktivkohle 1,5 Stunden anter Wasserstof geschüttelt. Der Katalysator wird über Kieselgur abfiltriert, mit Dichlormethan nachgewaschen und die organische Phase im Vakuum eingeengt. Man erhält 32 g rohes 15α-Ethyl-5-androsten-3β,17β-diol.

g) 3,9 g 15α-Ethyl-5-androsten-3β,17β-diol in 50 ml Pyridin werden mit 25 ml Essigsäureanhydrid 17 Stunden bei 20 °C gerührt. Dann wird mit salzsaurem Sole/Eiswasser gefällt, abfiltriert, mit Wasser gewaschen, in Dichlormethan aufgenommen, neutral gewaschen, über Natriumsulfat getrocknet und eingeengt. Nach der chromatographischen Reinigung an Kieselgel mit Hexan/Essigester erhält man 4,1 g 3,17-Diacetoxy-15α-ethyl-5-androsten.

h) 4,1 g 3β,17β-Diacetoxy-15α-ethyl-5-androsten in 62 ml Diethylether werden bei 0 °C mit 3,1 g N-Bromsuccinimid und mit einer Lösung aus 18 ml 70 %iger Perchlorsäure und 44 ml Wasser versetzt und 0,5 Stunden gerührt. Dann wird mit Natriumsulfatlösung versetzt, mit Essigester extrahiert, neutral gewaschen, über Natriumsulfat getrocknet und im Vakuum eingeengt. Man erhält 5,0 g rohes 3β,17β-Diacetoxy-5α-brom-15α-ethyl-6β-androstanol.

i) 5,0 g 3β,17β-Diacetoxy-5α-brom1-5α-ethyl-6β-androstanol in 175 ml Cyclohexan werden mit 2,5 g Bleitetracetat und 1,6 g Jod 2,5 Stunden am Rückfluß gesiedet. Dann wird der Feststoff abfiltriert, mit Essigester gewaschen, das Filtrat mit Natriumthiosulfat, Wasser und Natriumchloridlösung gewaschen, über Natriumsulfat getrocknet und im Vakuum eingeengt. Man erhält 4,8 g rohes 3β,17β-Diacetoxy-5α-brom-15α-ethyl-6β,19-oxidoandrostan.

j) 4,8 g rohes 3β,17β-Diacetoxy-5α-brom-15α-ethyl-6β,19-oxidoandrostan in 80 ml Eisessig werden mit 9,6 g Zink 3 Stunden bei 20 °C gerührt. Dann wird das Zink abgenutscht, mit Essigester gewaschen, mit Natriumhydrogencarbonat- und Natriumchloridlösung neutral gewaschen, über Natriumsulfat getrocknet und eingeengt. Nach der chromatographischen Reinigung an Kieselgel mit Hexan/Essigester erhält man 950 mg reines 3β,17β-Diacetoxy-15α-ethyl-19-hydroxy-5-androsten als Schaum.

k) 6,24 g 3β,17β-Diacetoxy-15α-ethyl-19-hydroxy-5-androsten in 62 ml Tetrahydrofuran werden mit 6,24 ml Dihydropyran und 31,2 mg p-Toluolsulfonsäure 1,5 Stunden bei 20 °C gerührt. Dann wird 1 ml Pyridin zugesetzt, mit Essigester verdünnt, mit Natriumhydrogencarbonat- und Natriumchloridlösung gewaschen, über Natriumsulfat getrocknet und im Vakuum eingeengt. Nach der chromatographischen Reinigung an Kieselgel mit Hexan/Essigester erhält man 5,1 g reines 3β,17β-Diacetoxy-15α-ethyl-19-(tetrahydropyran-2-yloxy)-5-androsten als Schaum.

l) 5 g 3β,17β-Diacetoxy-15α-ethyl-19-(tetrahydropyran-2-yloxy)-5-androsten werden mit 200 ml einer 0,2n methanolischen Kaliumhydroxydlösung 3 Stunden bei 20 °C gerührt. Dann wird mit 10 %iger Essigsäurelösung neutralisiert, die Lösung auf ein Drittel eingeengt, in Eiswasser/Kochsalz gefällt, abfiltriert, gewaschen, in Dichlormethan aufgenommen, neutral gewaschen, über Natriumsulfat getrocknet und eingeengt. Man erhält 3,94 g 15α-Ethyl-19-(tetrahydropyran-2-yloxy)-5-androsten-3β,17β-diol vom Schmelzpunkt 163 - 164 °C.

m) 3,54 g 15α-Ethyl-19-(tetrahydropyran-2-yloxy)-5-androsten-3β,17β-diol werden in 285 ml Toluol suspendiert und mit 106 ml Cyclohexanon und 3,54 g Aluminiumtriisopropylat 5 Stunden am Wasserabscheider gesiedet. Dann wird im Vakuum eingeengt, Kaliumnatriumtartratlösung zugefügt, 1 Stunde mit Wasserdampf destilliert, dreimal mit Dichlormethan extrahiert, neutral gewaschen, über Natriumsulfat getrocknet und im Vakuum zur Trockne eingeengt. Man erhält nach Chromatographie 2,69 g 15α-Ethyl-19-(tetrahydropyran-2-yloxy)-4-androsten-3,17-dion als Schaum.

n) 2,64 g 15α-Ethyl-19-(tetrahydropyran-2-yloxy)-4-androsten-3,17-dion in 66 ml Aceton werden mit 3,3 ml halbkonzentrierter Chlorwasserstoffsäure 5,5 Stunden bei 20 °C gerührt. Dann wird mit gesättigter Natriumhydrogencarbonatlösung neutralisiert, mit Wasser verdünnt, dreimal mit Dichlormethan extrahiert, neutral gewaschen, über Natriumsulfat getrocknet und im Vakuum eingeengt. Man erhält 2,652 g Rohprodukt und nach der chromatographischen Reinigung an Kieselgel mit Dichlormethan/Aceton 1 g reines 15α-Ethyl-19-hydroxy-4-androsten-3,17-dion vom Schmelzpunkt 123 - 125 °C.

2.) 15β-Ethyl-19-hydroxy-4-androsten-3,17-dion

50 g rohes 15β-Cyano-3β-hydroxy-5-androsten-17-on werden in 950 ml Tetrahydrofuran bei -20 °C mit 950 ml Diisobutylaluminiumhydrid-Lösung (1,2 M in Toluol) 2,5 Stunden gerührt. Dann wird mit verdünnter Schwefelsäure versetzt, über Kieselgur abgesaugt, die organische Phase mit Natriumhydrogencarbonatlösung neutral gewaschen, über Natriumsulfat getrocknet und im Vakuum eingeengt. Es werden 50,1 g roher 3β,17β-Dihydroxy-5-androsten-15β-carbaldehyd erhalten.

Die weitere Herstellung zum 15β-Ethyl-19-hydroxy-4-androsten-3,17-dion erfolgt analog der Herstellung der Ausgangsverbindungen 1 e) - n).

**Beispiel 1**

3-Chlormethylen-19-methylthio-4-androsten-17-on

500 mg 19-Methylthio-4-androsten-3,17-dion (Europäische Patentschrift 0 100 566) werden bei 0 °C zu einer Suspension von 5,21 g Chlormethyltriphenylphosphoniumchlorid in 50 ml Tetrahydrofuran, die mit 9,5 ml (1,6 m Buthyllithium in Ether) 0,5 Stunden bei 20 °C gerührt wurde, gegeben und 30 Minuten bei 0 °C gerührt. Dann wird mit Wasser versetzt, dreimal mit Essigester extrahiert, neutral gewaschen, über Natriumsulfat getrocknet und zur Trockne eingeengt. Man erhält 995 mg Rohprodukt, das nach der chromatographischen Reinigung 273 mg reines 3-Chlormethylen-19-methylthio-4-androsten-17-on vom Schmelzpunkt 114 - 115 °C,
$[\alpha]_D$ +241,5° ($CHCl_3$), als reine E-Form ergibt.

**Beispiel 2**

3-Fluormethylen-19-methylthio-4-androsten-17-on

500 mg 19-Methylthio-4-androsten-3,17-dion (Europäische Patentschrift 0 100 566) in 10 ml Dimethoxyethan werden bei 0 °C zu einer Suspension von 5,73 g Fluormethyltriphenylphosphoniumtetrafluoroborat in 40 ml Dimethoxyethan, die mit 9,5 ml Butyllithiumlösung (1,6 m in Ether) 0,5 Stunden gerührt wurde, tropfenweise hinzugefügt und 15 Minuten nachgerührt. Dann wird gesättigte Ammoniumchloridlösung zugegeben, mit Wasser verdünnt, dreimal mit Essigester extrahiert, neutral gewaschen, über Natriumsulfat getrocknet und im Vakuum eingeengt. Man erhält nach Chromatographie an Kieselgel mit Hexan/Essigester 331 mg reines 3-Fluormethylen-19-methylthio-4-androsten-17-on vom Schmelzpunkt 146 - 147 °C
$[\alpha]_D$ +288.4° ($CHCl_3$) als E:Z-Gemisch.

**Beispiel 3**

15α-Ethyl-3-methylen-19-hydroxy-4-androsten-17-on

2,7 g 15α-Ethyl-19-hydroxy-4-androsten-3,17-dion in 25 ml Tetrahydrofuran werden zu einer Suspension von 28,6 g Methyltriphenylphosphoniumbromid in 200 ml Tetrahydrofuran, die mit 200 ml Butyllithiumlösung (1,6 m in Ether) 1,5 Stunden bei 20 °C gerührt wurde, zugetropft und 1 Stunde bei 20 °C gerührt. Dann wird mit Wasser versetzt, dreimal mit Essigester extrahiert, neutral gewaschen, über Natriumsulfat getrocknet und im Vakuum vom Lösungsmittel befreit. Nach der chromatographischen Reinigung an Kieselgel mit Hexan/Essigester erhält man 1.95 g reines 15α-Ethyl-3-methylen-19-hydroxy-4-androsten-17-on als Schaum.
UV: $\epsilon_{239}$ = 13670.

**Beispiel 4**

15β-Ethyl-3-methylen-19-hydroxy-4-androsten-17-on

Die Herstellung erfolgt durch Reaktion von 15β-Ethyl-19-hydroxy-4-androsten-3,17-dion mit Methyltriphenylphosphoniumbromid analog Beispiel 3. Man erhält 15β-Ethyl-3-methylen-19-hydroxy-4-androsten-17-on als Schaum.
UV: $\epsilon_{239}$ = 13700.

**Beispiel 5**

3-Fluormethylen-4-androsten-17β-ol

17,2 g Fluormethyltriphenylphosphoniumtetrafluorborat werden in 150 ml Dioxan suspendiert und bei 25 °C portionsweise mit 6,74 g Kalium-tert.-butylat versetzt. Man läßt eine halbe Stunde bei 25 °C weiterrühren, gibt 3 g Testosteron hinzu und läßt weitere 30 Minuten bei 25 °C rühren. Dann wird die Reaktionsmischung auf Wasser gegeben, dreimal mit Dichlormethan extrahiert, die organischen Phasen neutral gewaschen, über Natriumsulfat getrocknet und im Vakuum vom Lösungsmittel befreit. Nach der Chromatographie an Kieselgel mit Hexan/Essigester erhält man 623 mg (E)-3-Fluormethylen-4-androsten-17β-ol vom Schmelzpunkt 138 - 140 °C, $X_1$, neben 1,24 g (Z)-3-Fluormethylen-4-androsten-17β-ol vom Schmelzpunkt 125 - 126 °C, $X_2$,
$X_1 = [\alpha]_D$ +148,4° ($CHCl_3$), $X_2 = [\alpha]_D$ +177,4° ($CHCl_3$).

(E)-3-Fluormethylen-4-androsten-17-on

623 mg (E)-3-Fluormethylen-4-androsten-17β-ol werden in 25 ml Toluol gelöst, mit 7 ml Cyclohexanon und dreimal mit 300 mg Aluminiumtriisopropylat bei 160 °C Badtemperatur am Wasserabscheider versetzt. Nach dem Abkühlen wird mit Kaliumnatriumtartrat 30 Minuten bei 60 °C gerührt, mit Dichlormethan verdünnt, neutralgewaschen, über Natriumsulfat getrocknet und eingeengt. Nach der Chromatographie an Kieselgel mit Hexan/Essigester erhält man 412 mg (E)-3-Fluormethylen-4-androsten-17-on vom Schmelzpunkt 142 - 143 °C,
$[\alpha]_D$ +235,8° ($CHCl_3$).

**Beispiel 6**

(Z)-3-Fluormethylen-4-androsten-17-on

1200 mg (Z)-3-Fluormethylen-4-androsten-17$\beta$-ol werden in 30 ml Aceton gelöst und bei 0 °C mit 1,5 ml Jones-Reagenz versetzt. Dann wird auf Wasser gegeben, dreimal mit Dichlormethan extrahiert, neutralgewaschen, über Natriumsulfat getrocknet und eingeengt. Nach der Chromatographie an Kieselgel mit Hexan/Essigester erhält man 842 mg (Z)-3-Fluormethylen-4-androsten-17-on vom Schmelzpunkt 101 - 102 °C,
$[\alpha]_D$ +246,5° ($CHCl_3$).

**Beispiel 7**

3-Fluormethylen-19-mercapto-4-androsten-17-on

17,2 g Fluormethyltriphenylphosphoniumtetrafluorborat werden in 120 ml Tetrahydrofuran suspendiert und bei 25 °C portionsweise mit 7,8 g Kalium-tert.-butylat versetzt. Man läßt 1 Stunde bei 25 °C weiterrühren, kühlt auf 0 °C und gibt 1,5 g 19-Mercapto-4-androsten-3,17-dion (Europäische Patentschrift 0 100 566) in 10 ml Tetrahydrofuran tropfenweise zu und läßt weitere 30 Minuten bei 0 °C rühren. Dann wird mit 1n-Salzsäure neutralisiert, die Reaktionsmischung auf Wasser gegeben, 4 mal mit Essigester extrahiert, die organischen Phasen neutral gewaschen, über Natriumsulfat getrocknet und im Vakuum vom Lösungsmittel befreit. Nach der Chromatographie an Kieselgel mit Hexan/Essigester erhält man 752 mg 3-Fluormethylen-19-mercapto-4-androsten-17-on vom Schmelzpunkt 118 - 120 °C.

## Patentansprüche

**1.** 3-Methylen-4-androsten-17-one der allgemeinen Formel I

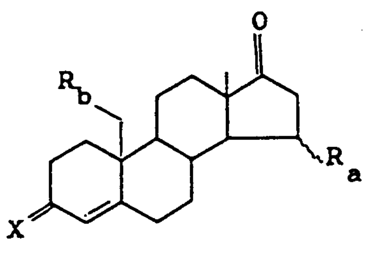

(I)

worin

- $R_a$ ein Wasserstoffatom oder einen gesättigten oder ungesättigten, gerad- oder verzweigtkettigen, gegebenenfalls substituierten Alkylrest mit 1 - 6 Kohlenstoffatomen, wobei $R_a$ in der $\alpha$- oder $\beta$-Position steht, und
- $R_b$ ein Wasserstoffatom, eine Hydroxyl- oder eine -$S(O)_nR_c$-Gruppe, bei der $R_c$ ein Wasserstoffatom oder eine Alkyl- oder Acylgruppe mit 1 - 4 Kohlenstoffatomen, n = 0, 1 oder 2 ist, und
- X $CH_2$, CHF, CHCl oder CHBr

bedeuten, wobei X nicht $CH_2$ ist, wenn $R_a$ Wasserstoff und $R_b$ Hydroxyl oder $R_a$ und $R_b$ jeweils Wasserstoff
bedeuten.

**2.** 3-Chlormethylen-19-methylthio-4-androsten-17-on.

**3.** 3-Fluormethylen-19-methylthio-4-androsten-17-on.

**4.** 15$\alpha$-Ethyl-3-methylen-19-hydroxy-4-androsten-17-on.

**5.** 15$\beta$-Ethyl-3-methylen-19-hydroxy-4-androsten-17-on.

**6.** (E)-3-Fluormethylen-4-androsten-17-on, (Z)-3-Fluormethylen-4-androsten-17-on.

**7.** 3-Fluormethylen-19-mercapto-4-androsten-17-on.

**8.** Pharmazeutische Präparate, gekennzeichnet durch den Gehalt an einer Verbindung gemäß Anspruch 1 - 7.

**9.** Verwendung der Verbindungen gemäß Anspruch 1 - 7 zur Herstellung von Präparaten zur Behandlung von östrogen-bedingten Krankheiten.

**10.** Verfahren zur Herstellung von 3-Halogenmethylen-4-androsten-17-onen der allgemeinen Formel I

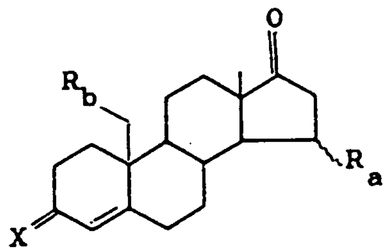

(I),

worin

$R_a$ ein Wasserstoffatom oder einen gesättigten oder ungesättigten gerad- oder verzweigtkettigen, gegebenenfalls substituierten Kohlenwasserstoffrest mit 1 - 6 Kohlenstoffatomen, wobei $R_a$ in der a- oder $\beta$-Position steht, und

$R_b$ ein Wasserstoffatom, eine Hydroxyl- oder eine -$S(O)_nR_c$-Gruppe, bei der $R_c$ ein Wasserstoffatom oder eine Acylgruppe mit 1 - 4 Kohlenstoffatomen und n = 0 oder $R_c$ eine Alkylgruppe mit 1 - 4 Kohlenstoffatomen und n = 0, 1 oder 2 ist, und

X $CH_2$, CHF, CHCl oder CHBr

bedeuten, wobei X nicht $CH_2$ ist, wenn $R_a$ Wasserstoff und $R_b$ Hydroxyl oder $R_a$ und $R_b$ jeweils Wasserstoff

bedeuten, dadurch gekennzeichnet, daß man

eine Verbindung der allgemeinen Formel II

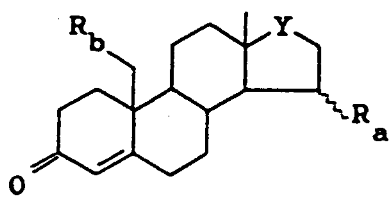

(II),

worin

$R_a$ ein Wasserstoffatom oder einen gesättigten oder ungesättigten, gerad- oder verzweigtkettigen, gegebenenfalls substituierten Kohlenwasserstoffrest mit 1 - 6 Kohlenstoffatomen, wobei $R_a$ in der $\alpha$- oder $\beta$-Position steht, und

$R_b$ ein Wasserstoffatom, eine Hydroxyl- oder eine -$S(O)_nR_c$-Gruppe, bei der $R_c$ ein Wasserstoffatom oder eine Acylgruppe mit 1 - 4 Kohlenstoffatomen und n = 0 oder $R_c$ eine Alkylgruppe mit 1 - 4 Kohlensstoffatomen und n = 0, 1 oder 2 ist und

Y eine Carbonyl- oder Hydroxymethylengruppe

bedeuten, mit einem Phosphorylid der allgemeinen Formel III

$(R_d)_3P = X$ (III),

worin

X — $CH_2$, CHF, CHCl oder CHBr, wobei X nicht $CH_2$ ist, wenn $R_a$ Wasserstoff und $R_b$ Hydroxyl oder $R_a$ und $R_b$ jeweils Wasserstoff bedeuten und

$R_d$ — einen gesättigten oder ungesättigten oder aromatischen Kohlenwasserstoffrest mit 1 - 10 Kohlenstoffatomen

bedeuten, umsetzt
und gegebenenfalls oxidiert.

## Claims

**1.** 3-methylene-4-androsten-17-ones of the general formula I

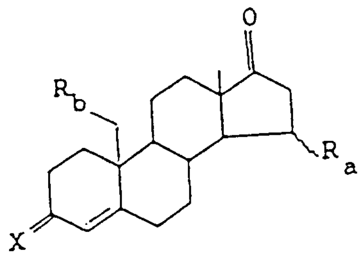

(I)

wherein

$R_a$ represents a hydrogen atom or a saturated or unsaturated, straight-chain or branched-chain, optionally substituted hydrocarbon radical having from 1 to 6 carbon atoms, $R_a$ being in the $\alpha$- or $\beta$-configuration, and

$R_b$ represents a hydrogen atom, a hydroxy group or a $-S(O)_nR_c$ group in which $R_c$ represents a hydrogen atom or an acyl group having from 1 to 4 carbon atoms and n = 0, or $R_c$ represents an alkyl group having from 1 to 4 carbon atoms and n = 0, 1 or 2, and

X represents $CH_2$, CHF, CHCl or CHBr,

but X does not represent $CH_2$ when $R_a$ represents hydrogen and $R_b$ represents hydroxy or when each of $R_a$ and $R_b$ represents hydrogen.

**2.** 3-chloromethylene-19-methylthio-4-androsten-17-one.

**3.** 3-fluoromethylene-19-methylthio-4-androsten-17-one.

**4.** 15$\alpha$-ethyl-3-methylene-19-hydroxy-4-androsten-17-one.

**5.** 15$\beta$-ethyl-3-methylene-19-hydroxy-4-androsten-17-one.

**6.** (E)-3-fluoromethylene-4-androsten-17-one,
(Z)-3-fluoromethylene-4-androsten-17-one.

**7.** 3-fluoromethylene-19-mercapto-4-androsten-17-one.

**8.** Pharmaceutical preparations characterised in that they contain a compound according to claims 1 to 7.

**9.** The use of the compounds according to claims 1 to 7 in the manufacture of preparations for the treatment of disorders caused by oestrogen.

**10.** Process for the manufacture of 3-halomethylene-4-androsten-17-ones of the general formula I

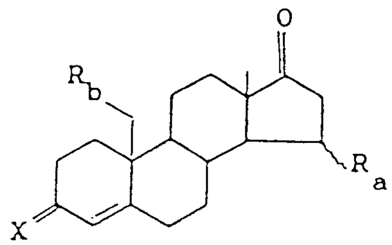

(I)

wherein

$R_a$ represents a hydrogen atom or a saturated or unsaturated, straight-chain or branched-chain, optionally substituted hydrocarbon radical having from 1 to 6 carbon atoms, $R_a$ being in the α- or β-configuration, and

$R_b$ represents a hydrogen atom, a hydroxy group or a $-S(O)_nR_c$ group in which $R_c$ represents a hydrogen atom or an acyl group having from 1 to 4 carbon atoms and n = 0, or $R_c$ represents an alkyl group having from 1 to 4 carbon atoms and n = 0, 1 or 2, and

X represents $CH_2$, CHF, CHCl or CHBr,

but X does not represent $CH_2$ when $R_a$ represents hydrogen and $R_b$ represents hydroxy or when each of $R_a$ and $R_b$ represents hydrogen,

characterised in that

a compound of the general formula II

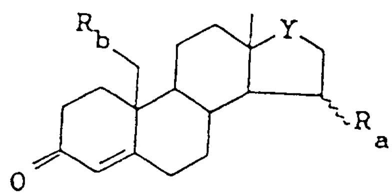

(II),

wherein

$R_a$ represents a hydrogen atom or a saturated or unsaturated, straight-chain or branched-chain, optionally substituted hydrocarbon radical having from 1 to 6 carbon atoms, $R_a$ being in the α- or β-configuration, and

$R_b$ represents a hydrogen atom, a hydroxy group or a $-S(O)_nR_c$ group in which $R_c$ represents a hydrogen atom or an acyl group having from 1 to 4 carbon atoms and n = 0, or $R_c$ represents an alkyl group having from 1 to 4 carbon atoms and n = 0, 1 or 2, and

Y represents a carbonyl or hydroxymethylene group,

is reacted with a phosphorylide of the general formula III

$(R_d)_3P = X$ (III),

wherein

X represents $CH_2$, CHF, CHCl or CHBr, but does not represent $CH_2$ when $R_a$ represents hydrogen and $R_b$ represents hydroxy or when each of $R_a$ and $R_b$ represents hydrogen, and

$R_d$ represents a saturated or unsaturated or aromatic hydrocarbon radical having from 1 to 10 carbon atoms,

and is optionally oxidised.

## Revendications

**1.** 3-Méthylène-4-androstène-17-ones de formule générale I ci-dessous :

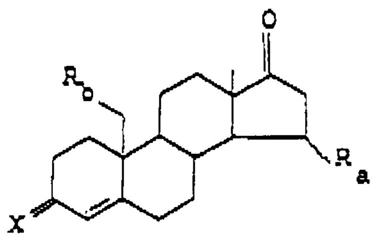

(I)

dans laquelle :

$R_a$ désigne un atome d'hydrogène ou un radical hydrocarboné ayant de 1 à 6 atomes de carbone, saturé ou insaturé, et à chaîne linéaire ou ramifiée, avec éventuellement des substituants, $R_a$ étant à la position $\alpha$ ou $\beta$,

$R_b$ un atome d'hydrogène, un hydroxyle ou un groupe $-S(O)_nR_c$, $R_c$ étant un atome d'hydrogène ou un groupe acyle ayant de 1 à 4 atomes de carbone et n le nombre 0, ou bien $R_c$ étant un alkyle en $C_{1-4}$ et n le nombre 0, 1 au 2, et

X un groupe $CH_2$, CHF, CHCl ou CHBr,

mais X n'étant pas le groupe $CH_2$ si $R_a$ est l'hydrogène et $R_b$ un hydroxyle ou encore si $R_a$ et $R_b$ sont chacun l'hydrogène.

**2.** 3-Chlorométhylène-19-méthylthio-4-androstène-17-one.

**3.** 3-Fluorométhylène-19-méthylthio-4-androstène-17-one.

**4.** 15$\alpha$-Ethyl-3-méthylène-19-hydroxy-androstène-17-one.

**5.** 15$\beta$-Ethyl-3-méthylène-19-hydroxy-androstène-17-one.

**6.** (E)-3-Fluorométhylène-4-androstène-17-one,
(Z)-3-Fluorométhylène-4-androstène-17-one.

**7.** 3-Fluorométhylène-19-mercapto-4-androstène-17-one.

**8.** Préparations pharmaceutiques caractérisées en ce qu'elles contiennent un composé des revendications 1 à 7 précédentes.

**9.** Emploi des composés selon les revendications 1 à 7 pour la fabrication de préparations destinées au traitement de maladies provoquées par des oestrogènes.

**10.** Procédé de préparation de 3-halogéno-méthylène-4-androstène-17-ones de formule générale I ci-dessous :

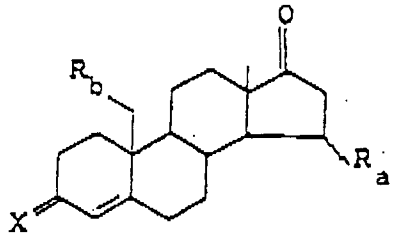

(I),

dans laquelle :

$R_a$ désigne un atome d'hydrogène ou un radical hydrocarboné ayant de 1 à 6 atomes de carbone, saturé ou insaturé, et à chaîne linéaire ou ramifiée, avec éventuellement des substituants, $R_a$ étant à la position $\alpha$ ou $\beta$,

$R_b$ un atome d'hydrogène, un hydroxyle ou un groupe $-S(O)_nR_c$, $R_c$ étant un atome d'hydrogène ou un groupe acyle ayant de 1 à 4 atomes de carbone et n le nombre 0, ou bien $R_c$ étant un alkyle en $C_{1-4}$ et n le nombre 0, 1 ou 2, et

X un groupe $CH_2$, CHF, CHCl ou CHBr,

mais X n'étant pas le groupe $CH_2$ si $R_a$ est l'hydrogène et $R_b$ un hydroxyle ou encore si $R_a$ et $R_b$ sont chacun l'hydrogène, procédé caractérise en ce que l'on fait réagir un composé de formule générale II :

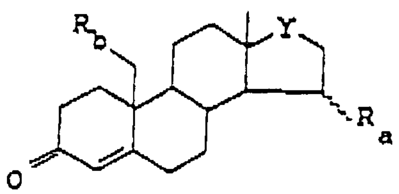

(II),

dans laquelle :

$R_a$ désigne l'hydrogène ou un radical hydrocarboné saturé ou insaturé ayant de 1 à 6 atomes de carbone, à chaîne linéaire ou ramifiée, et pouvant avoir éventuellement des substituants, $R_a$ étant à la position $\alpha$ ou $\beta$,

$R_b$ un atome d'hydrogène, un hydroxyle ou un groupe $-S(O)_nR_c$, dans lequel $R_c$ est un atome d'hydrogène ou un groupe acyle en $C_{1-4}$ et n = 0, ou bien $R_c$ étant un alkyle en $C_{1-4}$ et n = 0, 1 ou 2, et

Y représente un groupe carbonyle ou hydroxyméthylène,

avec un phosphorylide de formule générale III

$(R_d)_3P = X$ (III),

dans laquelle

X désigne un groupe $CH_2$, CHF, CHCl ou CHBr, mais X n'est pas le groupe $CH_2$ si $R_a$ est l'hydrogène et $R_b$ un hydroxyle ou bien si $R_a$ et $R_b$ sont chacun l'hydrogène, et

$R_d$ un radical hydrocarboné saturé ou insaturé ou aromatique pouvant avoir de 1 à 10 atomes de carbone,

et le cas échéant on procède à une oxydation.